# EUROPEAN PATENT SPECIFICATION

(11) **EP 0 538 402 B1**
(45) Date of publication and mention of the grant of the patent: **08.03.2006**
(21) Application number: 91915046.6
(22) Date of filing: 02.07.1991
(51) Int. Cl.: A61K 51/04, A61K 51/12, C07B 59/00

(54) **METHOD OF PREPARING A RADIOACTIVE RHENIUM COMPLEX SOLUTION**
METHODE ZUR HERSTELLUNG EINER LÖSUNG AUS RADIOAKTIVEN RHENIUMKOMPLEXEN
PROCEDE DE PREPARATION D'UNE SOLUTION D'UN COMPLEXE DE RHENIUM RADIOACTIF

(30) Priority: 06.07.1990 EP 90201817
(43) Date of publication of application: 28.04.1993
(73) Proprietor: MALLINCKRODT INC., St. Louis, MO 63134 (US)
(72) Inventor: PANEK-FINDA, Helena, NL-1380 AC Weesp (NL)
(74) Representative: Van Someren, Petronella F. H. M.
(86) International application number: PCT/US1991/004704
(87) International publication number: WO 1992/000758

(56) References cited:
- EP-A- 0 250 966
- EP-A- 0 300 431
- WO-A-90/02571
- WO-A-90/13530
- US-A- 4 839 467
- US-A- 4 925 925
- Radiology, Volume 166, issued 1988, (US), H. MAXON et al., "RE-186(SN) Heop for treatment of multiple metastic fuci in bare: human biodistribution and dosimetric studies", see pages 501-507.
- DEUTSCH E. ET AL: 'The Chemistry of Rhenium and Technetium as Related to the USe of Isotopes of these Elements in Therapeutic and Diagnostic Nuclear Medicine' NUCL. MED. BIOL. vol. 13, no. 4, 1986, pages 465 - 477

## Description

The invention relates to a method of preparing a solution of a radioactive rhenium complex, by reacting at elevated temperature a radioactive perrhenate in an aqueous solution with a ligand in the presence of a reductant and optionally an antioxidant under anaerobic conditions. Such a preparation method has been described by Deutsch et al, viz in U.S. patent 4778672, in European patent application 250966 and in Nucl. Med. Biol. 13, 1986, 465-477. US-4,839,467 describes the preparation of radioactive rhenium complexes under anaerobic conditions by reacting an aqueous solution of perrhenate with 2-hydroxy butyric acid and a reductant at temperatures of 60-100°C. In particular in their above mentioned publication, Deutsch and coworkers have explained in detail the differences between the preparation of rhenium complexes and of technetium complexes and the practical consequences thereof in nuclear medicine applications. Re complexes are more stable in their higher oxidation states than are analogous Tc complexes. Consequently reduced Re radiopharmaceuticals will tend to be re-oxidized back to perrhenate. This instability is a serious disadvantage in the use of Re radiopharmaceuticals because the impurities, formed during preparation and storage of these pharmaceuticals, such as uncomplexed perrhenate and rhenium dioxide, form a serious radiation burden for various organs and tissues, such as kidneys, liver and blood. As a matter of fact, radioactive labelled rhenium compounds are intended for combating or controlling tumors or can be used as palliation agents for the pain caused by certain malignant tumors. It will therefore be evident, that such radiolabelled compounds which are intended, for example, for damaging tumors, are highly injurious to the health of the patient if arrived in a wrong place of the body.

Deutsch et al have recognized the above problems and they have proposed a variety of measures to counter these problems. First, they have proposed to perform the preparation of the desired complex and to store said complex under substantially anaerobic conditions, to add to said complex an antioxidant and to use for the preparation a large excess of reductant, thus requiring a large amount of ligand. Second, they have proposed to purify the complex solution by a chromatographic procedure just prior to use, which they consider as most effective. Such a chromatographic procedure involves rather complicated manipulation of highly radioactive material, such as loading the prepared column with the radioactive solution to be purified, elution and collection of the individual fractions, and selection and combining of the proper fractions comprising the purified rhenium complex. The purified rhenium complex must be administered to the patient within one hour of preparation to avoid degradation. Therefore this laborious purification method involving the manipulation of highly radioactive material should be performed in the clinic or clinical hospital where the radiopharmaceutical is to be used.

It is the object of the present invention to provide a method of preparing a solution of a radioactive rhenium complex as defined in the opening paragraph, in which such a laborious purification just prior to use is not necessary. Further, it is the object of the present invention to provide a method of preparing a rhenium-comprising radiopharmaceutical with an improved stability.

This object can be achieved by reacting at elevated temperature a radioactive perrhenate in a aqueous solution with a ligand in the presence of a reductant and optionally an antioxidant under anaerobic conditions, characterized in that said reaction is carried out at a pH from 1.5 to 5 and by heating the reaction components for at least 10 minutes at a temperature of over 100°C.

Surprisingly it has been found, that under the above reactions conditions a product is obtained which is sufficiently stable to be stored and transported to the user, i.e. the clinic or clinical laboratory, without objectionable deterioration of the radioactive rhenium complex. Consequently a purification of the product prior to use is not necessary. In addition it has been found, that the product obtained in this manner presents a superior biological behaviour in comparison with the product prepared according to the known method.

The preparation of the solution of the radioactive rhenium complex can be carried out conveniently by the producer in a reaction vessel suitable for performing reactions under elevated pressure, for example in an autoclave. Preferably the method of the invention is carried out in such manner, that a radioactive perrhenate in a substantially aqueous solution, having a concentration of rhenium in said solution within the range of 5 x 10⁻⁶ M to 2 x 10⁻³ M, is reacted with said ligand in the presence of said reductant, by mixing said solution with a solution or a lyophilized solid, comprising an excess of said reductant and of said ligand with respect to the quantity of rhenium, and by then heating said reaction mixture.

Of course the ligand to be used in the above complexing reaction is a ligand that complexes with rhenium. For the intended purpose of the radiopharmaceutical to be prepared a bone seeking ligand is preferred. Suitable bone seeking ligands are polyphosphates, pyrophosphates, phosphonates, diphosphonates, phosphonites and imidodiphosphates. The therapeutic radiopharmaceuticals are in particular intended for use as palliation agents for the pain caused by metastatic bone cancer. For this purpose are particularly effective rhenium complexes of diphosphonate ligands selected from 1-hydroxyethylidene -1,1-diphosphonic acid, hydroxymethylene diphosphonic acid, methylene diphosphonic acid, (diphosphonomethyl)-butanedionic acid, aminoethane diphosphonic acid, (dimethylamino)methyl diphosphonic acid, ethylenediamine tetra(methylene phosphonic acid) and propane-3-amino-1-hydroxy-1,1-diphosphonic acid, preferably 1-hydroxyethylidene-1,1-diphosphonic acid.

As regards the radiation characteristics, rhenium-186 and rhenium-188 are suitable radionuclides of rhenium for the above therapeutic application.

Also the pH of the solution during the complex formation may vary between certain limits, a pH between 2 and 2.5 is preferred to advance the formation of the desired rhenium complex.

It has been observed, that the best results are obtained when the above complex-forming reaction is carried out by heating the reaction components for at least 10 min. at a temperature of approx. 120°C. Then the rhenium complex obtained proves to have a very high resistance against degradation on ageing and a surprisingly enhanced biological performance, as will be apparent from the Examples.

The present invention also relates to a method of preparing a ready-to-inject product, i.e. a sterile radiopharmaceutical composition comprising a radioactive rhenium complex solution, wherein said solution is prepared as described hereinbefore and then the pH of the product obtained is adjusted to 5-6 by addition of a suitable buffer solution under an inert atmosphere, if desired while adding a pharmaceutically acceptable dilution liquid, after which the final radiopharmaceutical composition is sterilized by autoclaving at approx. 120° C and subsequently stored for a period of at least 3 hours before administration. Remarkably it has been observed, that said radiopharmaceutical composition is not in an optimum condition for administration to humans directly after preparation, but that a time period of at least 3 hours between autoclaving and administration is needed to obtain the desired radioactive rhenium complex in said composition having a high radiochemical purity. So apparently in this time period the desired rhenium complex is recovered. The thus at least 3-hours-aged radiopharmaceutical composition can be stored and transported to the user at ambient temperature without noticeable deterioration and can there be directly administered to a patient without any manipulations.

Alternatively said sterile radiopharmaceutical composition can be prepared by first preparing said radioactive rhenium complex as described hereinbefore and by then autoclaving the product obtained at approx. 120° C, after which the pH of the sterile product is adjusted to 4-9, preferably to 5-8, by addition of a suitable buffer solution under an inert atmosphere, if desired while adding a pharmaceutically acceptable dilution liquid. The composition thus obtained is immediately ready for use, i.e. for administration to a patient. The addition of the buffer solution should preferably be performed prior to administration, so preferably in the clinic or hospital. Because the operation of adding a buffer solution to the radioactive material is a so simple procedure, this method is not disadvantageous compared to the former method of preparing the radiopharmaceutical composition. Suitable buffers are pharmaceutically acceptable buffers, such as acetate buffer, citrate buffer, phosphate buffer, TRIS buffer and HEPES buffer. If the method of preparing the radioactive rhenium complex solution is performed by heating the reaction components at a temperature of approx. 120° C, which reaction temperature is preferred, the complexing reaction and the autoclaving can even be combined. In other words, in that case a separate autoclaving can be omitted.

Further the invention relates to the ready-for-use product, i.e. the sterile radiopharmaceutical composition comprising the product prepared according to the method as described above, and to the use of said composition for radotherapeutically treating a warm-blooded living being. For this purpose said composition is administered to said being in a quantity effective for combating or controlling tumors or for palliating the pain caused by certain metastatic tumors.

Finally the invention relates to a kit for preparing a sterile radiopharmaceutical composition according to the above alternative preparation method. As stated before, in performing said alternative method the pH of the final autoclaved product should be adjusted by adding a buffer solution, preferably prior to administration. Consequently, such a kit for preparing a sterile radiopharmaceutical composition for therapeutical application comprises (i) a sterile radioactive rhenium complex solution obtained as described above, and (ii) a buffer solution suitable for adjusting the pH of the solution defined sub (i) to 4-9, preferably to 5-8, to which solution, if desired, a pharmaceutically acceptable dilution liquid has been added.

The invention will now be described in more detail with reference to the following specific examples.

### Example I

### Preparation of rhenium-186 labelled 1-hydroxyethylidene diphosphonate (Re186-HEDP bulk)

Re186-radioisotope is obtained by irradiation of Re185-metal (97.4% enriched) at high flux of thermal neutrons in the nuclear reactor. The irradiated target material is processed to the final chemical form of NaReO₄ which is to be used as a starting radioactive material for preparation of Re186-HEDP complex.

The sodium perrhenate of a high chemical purity is obtained by oxidation of Re-metal with 30%-hydrogen peroxide followed by complete evaporation to dryness. The dry residue - free of hydrogen peroxide - is then dissolved in 0.9% sodium chloride aqueous solution and the radioactive bulk containing the Re186 - radioisotope in the form of sodium perrhenate is finally formulated to the solution, where the rhenium concentration lies between 10-200 ug/ml and the radioactive concentration is equal to expected precalibrated activity at the time of administration. Natural pH of this solution lies between 5 and 5.5. This way processed sodium perrhenate does not contain any intermediate products and contaminants. Sterility, apyrogenicity and absence of particulate matter is secured by bacterial filtration through Millex CS® filter.

HEDP-reaction mixture - is prepared by lyophilizing deaerated concentrated aqueous bulk solution of disodium 1-hydroxymethylidene-1,1-diphosphonate, gentisic acid (antioxidant) and tin(II)chloride dihydrate (reductant) in concentrations of 100, 30 and 35 mg per 1.5 ml respectively. The volume of the solution to be lyophilized is dependent on the size of the batch to be prepared. An amount of 0.15 ml of the bulk solution to be lyophilized is equal to one patient dosis.

Re186-HEDP bulk - is prepared by addition of equal volume (1 ml Na(Re186)O₄/one patient dosis) of the sterile Re186-bulk solution to the contents of the vial containing the HEDP-reaction mixture, avoiding contamination of the vial interior with air. After reconstitution of the lyophilized HEDP-reaction mixture in Na (Re186)O₄ solution under inert gas atmosphere, e.g. nitrogen, the radioactive solution is autoclaved for 10-30 minutes at 121° C.

The pH of the solution in the reaction vial is typically between 2-2.5. This solution typically contains more than 99% of the Re186-HEDP complex.

### Example II

### Preparation of a sterile radiopharmaceutical composition comprising Re186-HEDP

Re186-HEDP-Injection pH 5 - 5.5 -is prepared by mixing of the Re186-HEDP bulk, obtained according to Example I, with equal volume of deaerated sodium acetate buffer solution pH 7.5-9.5 under inert atmosphere. 2-ml portions of the pH-adjusted Re186-HEDP bulk are dispensed in the autoclavable transport vials which are than crimp-sealed under inert gas atmosphere and autoclaved. Typical pH of this solution is 5 - 5.5 and content of released 186ReO₄⁻ due to the autoclaving of Re186-HEDP complex at pH>5 might be as high as 7-8%. This solution is not in an optimum condition for administration to humans directly after preparation, but, a time period of at least 3 hours between autoclaving and administration is needed for recovery of the Re186-HEDP complex to approx. 99%. This way prepared radiopharmaceutical 3-hours-aged might be directly administered to a patient without further adjustments of pH or concentration.

### Example III

### Preparation of a sterile radiopharmaceutical composition comprising Re186-HEDP.

Re186-Injection pH 2.2-2.5 is prepared by dispensing of 1 ml of Re186-HEDP bulk under an inert atmosphere and autoclaving of the crimp-sealed vials. This acidic solution can be used at any time after autoclaving, when properly adjusted by addition of 1 ml acetate buffer prior administration to the patient. The radiochemical purity of Re 186-HEDP in this solution is typically ≥ 99%.

### Example IV

### Comparative experiments in recovery of Re 186-HEDP after autoclaving at adjusted pH under anaerobic and aerobic conditions

A bulk solution of Re186-HEDP prepared under anaerobic conditions and adjusted to pH 5.3 with acetate buffer is dispensed into vials under nitrogen and normal air atmosphere respectively. The vials contain approx. 400-600 MBq Re186. Both groups of test vials are autoclaved and submitted to consecutive radiochemical purity analysis at the time intervals from T₀ₕ-T₂₄ₕ. The results are compared with radiochemical purity of the Re 186-HEDP bulk prior to autoclaving. Results in Table A demonstrate the instability of the Re186-HEDP complex under aerobic conditions and also recovery of the Re186-HEDP to original radiochemical purity in time of storage under anaerobic condition. It can be observed, that heating of Re186-HEDP complex at pH which is unfavorable to the Re186-HEDP complex formation releases always some free 186ReO₄⁻. On the other hand, when the heating and storage of the complex is performed in absence of oxygen, the Re186-HEDP rather quickly recovers to the original state.

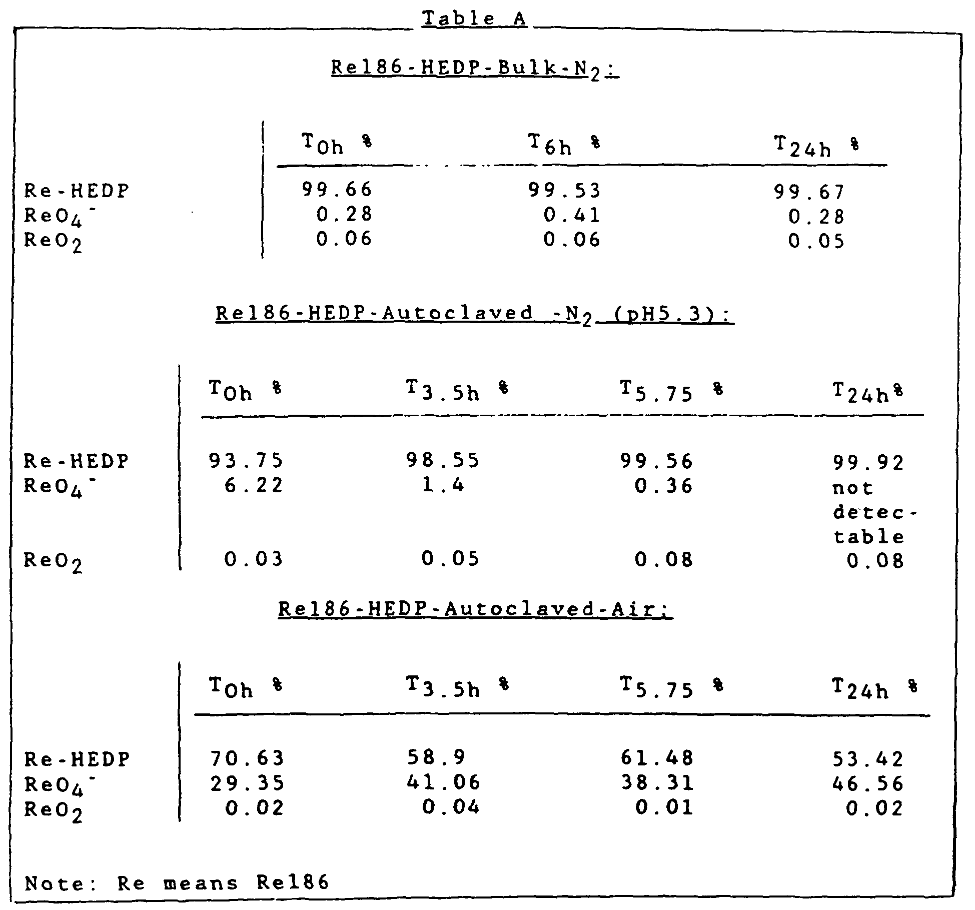

The influence of the reaction temperature on the stabillity of Re186-HEDP is demonstrated in a comparative experiment, where the labelling of HEDP with rhenium-186 is carried out as described in Example I, but now by heating for 15 minutes in a boiling water bath, i.e. at a temperature just below 100° C. It is observed that the product thus obtained gradually degrades during storage at ambient temperature, viz. from 99.12% at t-0 down to 93.72 % at t-24 h.

### Example V

When using the preparation methods as described in Examples I, II and III, the injectable compositions a-f are obtained as follows:
composition a:
   a. dissolution of 186ReO₄⁻ residue (isotope production) in reaction solution under an inert atmosphere.
   b. heating the reaction mixture at temperature > 100°C under inert atmosphere > 10 min.
   c. pH adjustment (acetate buffer) and dilution to apropiate volume
   d. dispensing of the Re186-HEDP pH > 5 into the vials
   e. autoclaving of the vials under an inert atmosphere
composition b:
   a. dissolution of 186ReO₄⁻ residue in reaction solution under an inert atmosphere
   b. adjustment of the volumic activity with the reaction solution
   c. dispensing of the reaction mixture (1 ml/vial) under an inert atmosphere
   d. autoclaving of the dispensed reaction mixture
   e. addition of 1 ml acetate buffer prior to administration
composition c:
   a. dissolution of lyophilized reaction mixture in Na(Re186) O₄⁻ solution under an inert atmosphere
   b. heating of the Re 186-reaction mixture for 10 or more minutes at temperature > 100°C
   c. adjustment of pH to 5-5.5 by addition of acetate buffer under inert atmosphere
   d. dispensing of the Re186-HEDP pH 5-5.5 (2 ml/vial) under an inert atmosphere
   e. autoclaving of the vials
composition d:
   a. dissolution of the lyophilized reaction mixture in Na (Re186)O₄ under an inert atmosphere
   b. dispensing of the Re186-reaction mixture under an inert atmosphere (1 ml/vial)
   c. autoclaving of the dispensed Re186-reaction mixture
   d. addition of acetate buffer (1 ml) to the Re186-HEDP prior to administration
composition e:
   a. dispensing of calibrated Na(Re186)O₄ into the vials containing a freeze-dried 1-patient dosis of the reaction mixture - under an inert atmosphere
   b. autoclaving of the vials
   c. addition of 1 ml citrate buffer prior to administration in the hospital
composition f:
   a. dispensing of calibrated Na(Re186)O₄ into the vials containing a freeze-dried 1-patient dosis of the reaction mixture - under an inert atmosphere
   b. autoclaving of the vials
   c. addition of 1 ml acetate buffer to each vial under an inert atmosphere
   d. autoclaving of the Re186-HEDP - pH 5 - 5.5

Compositions b, d and e can be delivered as two-component (vial) kits, a first vial containing a solution of the radioactive rhenium complex, and a second vial containing a buffer solution for adjusting the pH of the radiopharmaceutical prior to administration.

### Example VI

### Comparative biological experiment

Composition g and h are prepared identically, viz. by addition of 1 ml 186ReO₄⁻ to the lyophilized HEDP reaction mixture under anaerobic conditions and heating the reconstituted mixture in a boiling water bath for 15 minutes. After brief cooling, 1 ml of acetate buffer is added to each preparation respectively under nitrogen. Composition g is administered to the test animals within 0.5 hour after addition of acetate buffer, while composition h is administered after 24 hours of storage at room temperature.

Composition k and l are prepared batchwise, by addition of 5 ml 186ReO₄ to lyophilized reaction mixtures in fivefold quantities and autoclaving for 25 minutes at 121°C. 1-ml portions of composition k are dispensed into the vials, crimp-sealed and set aside for 24 hours storage at room temperature, to be adjusted with 1 ml of the acetate buffer prior to administration. On the other hand, 1-ml portions of composition l are immediately after dispensing adjusted by addition of 1 ml acetate buffer and autoclaved again (25 min. at 121°C), to be set aside at room temperature and administered 24 hours after preparation.
All operations are performed under anaerobic conditions.

4 Groups of Sprague-Dawley female rats are injected with the above Re186-HEDP composition g, h, k and l in order to compare the biological performance of these compositions.

Three hours after injection the test animals are sacrificed and the organ distribution is determined. From this organ distribution the bone/organ ratio is determined. The values in table B below are the average values of four test animals per group.

**Table B**

| composition | bone/organ ratio | | |
|---|---|---|---|
| | bone/blood | bone/kidney | bone/muscle |
| g | 17.469 | 1.937 | 208.847 |
| h | 20.464 | 2.097 | 234.336 |
| k | 27.594 | 3.184 | 302.360 |
| l | 27.766 | 3.373 | 338.217 |

The above results show, that the biological performance of the Re186-HEDP complex prepared by autoclaving at approx. 120°C is appreciably enhanced in comparison with the complex, prepared by heating in a boiling water bath.

## Claims

1. A method of preparing a solution of a radioactive rhenium complex, by reacting at elevated temperature a radioactive perrhenate in a aqueous solution with a ligand in the presence of a reductant and optionally an antioxidant under anaerobic conditions, **characterized in that** said reaction is carried out at a pH from 1.5 to 5 and by heating the reaction components for at least 10 minutes at a temperature of over 100°C.

2. A method as claimed in claim 1, **characterized in that** a radioactive perrhenate in a aqueous solution, having a concentration of rhenium in said solution within the range of approx. 5 x 10⁻⁶ M to approx. 2 x 10⁻³ M, is reacted with said ligand in the presence of said reductant, by mixing said solution with a solution or a lyophilized solid, comprising an excess of said reductant and of said ligand with respect to the quantity of rhenium, and by then heating said reaction mixture.

3. A method as claimed in claim 1 or 2, wherein the ligand is a bone seeking ligand.

4. A method as claimed in claim 3, wherein the ligand is a polyphosphate, pyrophosphate, phosphonate, diphosphonate, phosphonite or imidodiphosphate.

5. A method as claimed in claim 4, wherein the ligand is a diphosphonate selected from 1-hydroxyethylidene -1,1-diphosphonic acid, hydroxymethylene diphosphonic acid, methylene diphosphonic acid, (diphosphonomethyl)-butanedionic acid, aminoethane diphosphonic acid, (dimethylamino)methyl diphosphonic acid, ethylenediamine tetra(methylene phosphonic acid) and propane-3-amino-1-hydroxy-1,1-diphosphonic acid, preferably 1-hydroxyethylidene-1,1-diphosphonic acid.

6. A method as claimed in any of the preceding claims, wherein the radioactive perrhenate contains either rhenium-186 or rhenium-188.

7. A method as claimed in any of the preceding claims, **characterized in that** the reaction is carried out at a pH between 2 and 2.5.

8. A method as claimed in any of the preceding claims, **characterized in that** the reaction is carried out by heating said reaction components at a temperature of approx. 120° C.

9. A method of preparing a sterile radiopharmaceutical composition comprising a radioactive rhenium complex solution, **characterized in that** said radioactive rhenium complex solution is prepared according to any of the preceding claims and that then the pH of the product obtained is adjusted to 5-6 by addition of a suitable buffer solution under an inert atmosphere, if desired while adding a pharmaceutically acceptable dilution liquid, after which the final radiopharmaceutical composition is sterilized by autoclaving at approx. 120° C and subsequently stored for a period of at least 3 hours before administration.

10. A method of preparing a sterile radiopharmaceutical composition comprising a radioactive rhenium complex solution, **characterized in that** said radioactive rhenium complex solution is prepared according to any of claims 1-7 and that then the product obtained is autoclaved at approx. 120° C, after which the pH of the sterile product is adjusted to 4-9, preferebly to 5-8, by addition of a suitable buffer solution under an inert atmosphere, if desired while adding a pharmaceutically acceptable dilution liquid.

11. A method of preparing a sterile radiopharmaceutical composition comprising a radioactive rhenium complex solution, **characterized in that** said radioactive rhenium complex solution is prepared according to claim 8 and that then the pH of the product obtained is adjusted to 4-9, preferably to 5-8, by addition of a suitable buffer solution under an inert atmosphere, if desired while adding a pharmaceutically acceptable dilution liquid.

12. A sterile radiopharmaceutical composition for therapeutical application, comprising the product prepared according to the method as claimed in any of claims 9-11.

13. Use of a composition as claimed in claim 12 for the manufacture of a medicament for combating or controlling tumours or for palliating the pain caused by certain metastatic tumours.

14. A kit for preparing a sterile radiopharmaceutical composition for therapeutical application, comprising (i) a sterile radioactive rhenium complex solution obtained by performing the method as claimed in any of claim 1-7, followed by autoclaving the product thus obtained at approx. 120° C, or by performing the method as claimed in claim 8, and (ii) a buffer solution suitable for adjusting the pH of the solution defined sub (i) to 4-9, preferably to 5-8, to which solution, if desired, a pharmaceutically acceptable dilution liquid has been added.

## Patentansprüche

1. Verfahren zur Herstellung einer Lösung eines radioaktiven Rhenium-Komplexes, wobei bei erhöhter Temperatur ein radioaktives Perrhenat in einer wässrigen Lösung mit einem Liganden in Gegenwart eines Reduktionsmittels und wahlweise eines Antioxidationsmittels unter anaeroben Bedingungen umgesetzt wird, **dadurch gekennzeichnet, dass** die Reaktion bei einem pH von 1,5 bis 5 und durch Erhitzen der Reaktionskomponenten für wenigstens 10 Minuten bei einer Temperatur von über 100°C durchgeführt wird.

2. Verfahren nach Anspruch 1, **dadurch gekennzeichnet, dass** ein radioaktives Perrhenat in einer wässrigen Lösung, die eine Konzentration von Rhenium in der Lösung innerhalb eines Bereiches von etwa 5 x 10⁻⁶ M bis etwa 2 x 10⁻³ M aufweist, mit dem Liganden in Gegenwart des Reduktionsmittels umgesetzt wird, indem die Lösung mit einer Lösung oder einem lyophilisierten Feststoff gemischt wird, die/der in Bezug auf die Menge des Rheniums einen Überschuss des Reduktionsmittels und des Liganden umfasst, und danach die Reaktionsmischung erhitzt wird.

3. Verfahren nach Anspruch 1 oder 2, wobei der Ligand ein Ligand mit Affinität zu Knochen ist.

4. Verfahren nach Anspruch 3, wobei der Ligand ein Polyphosphat, Pyrophosphat, Phosphonat, Diphosphonat, Phosphonit oder Imidodiphosphat ist.

5. Verfahren nach Anspruch 4, wobei der Ligand ein Diphosphonat ist, das aus 1-Hydroxyethyliden-1,1-Diphosphonsäure. Hydroxymethylendiphosphonsäure, Methylendiphosphonsäure, (Diposphonmethyl)-butandiionische Säure, Aminoethandiphosphonsäure, (Dimethylamino)-methyldiphosphonsäure, Ethylendiamintetra (methylenphosphonsäure) und Pröpan-3-amino-1-hydroxy-1,1-diphosphonsäure, bevorzugt 1-Hydroxyethyliden-1,1-diphosphonsäure, ausgewählt wird.

6. Verfahren nach einem der vorherigen Ansprüche, wobei das radioaktive Perrhenat entweder Rhenium-186 oder Rhenium-188 enthält.

7. Verfahren nach einem der vorherigen Ansprüche, **dadurch gekennzeichnet, dass** die Reaktion bei einem pH zwischen 2 und 2,5 durchgeführt wird.

8. Verfahren nach einem der vorherigen Ansprüche, **dadurch gekennzeichnet, dass** die Reaktion durch Erhitzen der Reaktionskomponenten bei einer Temperatur von etwa 120°C durchgeführt wird.

9. Verfahren zur Herstellung einer sterilen radiopharmazeutischen Zusammensetzung, die eine radioaktive Rhenium-Komplexlösung umfasst, **dadurch gekennzeichnet, dass** die radioaktive Rhenium-Komplexlösung gemäß einem der vorherigen Ansprüche hergestellt wird, und dass dann der pH des erhaltenen Produktes durch Zugabe einer geeigneten Pufferlösung unter einer inerten Atmosphäre auf 5-6 eingestellt wird, falls gewünscht, unter Zugabe einer pharmazeutisch geeigneten Verdünnungsflüssigkeit, wonach die radiopharmazeutische Endzusammensetzung durch Autoklavieren bei etwa 120°C sterilisiert wird und nachfolgend über einen Zeitraum von wenigstens 3 Stunden vor der Verabreichung gelagert wird.

10. Verfahren zur Herstellung einer sterilen radiopharmazeutischen Zusammensetzung, die eine radioaktive Rhenium-Komplexlösung umfasst, **dadurch gekennzeichnet, dass** die radioaktive Rhenium-Komplexlösung gemäß einem der Ansprüche 1-7 hergesellt wird, und dass dann das erhaltene Produkt bei etwa 120°C autoklaviert wird, wonach der pH des sterilen Produktes durch Zugabe einer geeigneten Pufferlösung unter einer inerten Atmosphäre auf 4-9, bevorzugt auf 5-8 eingestellt wird, falls gewünscht, unter Zugabe einer pharmazeutisch geeigneten Verdünnungsflüssigkeit.

11. Verfahren zur Herstellung einer sterilen radiopharmazeutischen Zusammensetzung, die eine radioaktive Rhenium-Komplexlösung umfasst, **dadurch gekennzeichnet, dass** die radioaktive Rhenium-Komplexlösung nach Anspruch 8 hergestellt wird, und dass dann der pH des erhaltenen Produktes durch Zugabe einer geeigneten Pufferlösung unter einer inerten Atmosphäre auf 4-9, bevorzugt auf 5-8 eingestellt wird, falls gewünscht unter Zugabe einer pharmazeutisch geeigneten Verdünnungsflüssigkeit.

12. Eine sterile radiopharmazeutische Zusammensetzung zur therapeutischen Anwendung, die das Produkt, das gemäß dem Verfahren nach einem der Ansprüche 9-11 hergestellt wird, umfasst.

13. Verwendung einer Zusammensetzung nach Anspruch 12 zur Herstellung eines Arzneimittels zum Bekämpfen oder Kontrollieren von Tumoren oder zur Linderung der Beschwerden, die durch bestimmte Tumormetastasen verursacht werden.

14. Kit zur Herstellung einer sterilen radiopharmazeutischen Zusammensetzung zur therapeutischen Anwendung, das umfasst (i) eine sterile radioaktive Rhenium-Komplexlösung, die durch das Durchführen des Verfahrens nach einem der Ansprüche 1-7 erhalten wird, gefolgt durch das Autoklavieren des dabei erhaltenen Produktes bei etwa 120°C oder durch das Durchführen des Verfahrens nach Anspruch 8, und (ii) eine Pufferlösung, die zum Einstellen des pH der unter (i) definierten Lösung auf 4-9, bevorzugt auf 5-8, geeignet ist, wobei zur Lösung, falls gewünscht, eine pharmazeutisch geeignete Verdünnungslösung zugegeben wurde.

## Revendications

1. Procédé de préparation d'une solution d'un complexe de rhénium radioactif, par mise en réaction à température élevée d'un perrhénate radioactif dans une solution aqueuse avec un ligand en présence d'un réducteur et facultativement d'un antioxydant dans des conditions d'anaérobie, **caractérisé en ce que** ladite réaction est effectuée à un pH allant de 1,5 à 5, et par chauffage des composants de la réaction pendant au moins 10 minutes à une température supérieure à 100 °C.

2. Procédé selon la revendication 1, **caractérisé en ce qu'**un perrhénate radioactif dans une solution aqueuse, présentant une concentration de rhénium dans ladite solution variant d'approximativement 5 x 10⁻⁶ M à approximativement 2 x 10⁻³ M, est mis en réaction avec ledit ligand en présence dudit réducteur, par mélange de ladite solution avec une solution ou un solide lyophilisé comprenant un excès dudit réducteur et dudit ligand par rapport à la quantité de rhénium, et par chauffage ensuite du mélange réactionnel.

3. Procédé selon la revendication 1 ou 2, dans lequel le ligand est un ligand ostéotrope.

4. Procédé selon la revendication 3, dans lequel le ligand est un polyphosphate, un pyrophosphate, un phosphonate, un diphosphonate, un phosphonite ou un imidodiphosphate.

5. Procédé selon la revendication 4, dans lequel le ligand est un diphosphonate choisi parmi l'acide 1-hydroxyéthylidène-1,1 diphosphonique, l'acide hydroxyméthylène diphosphonique, l'acide méthylène diphosphonique, l'acide diphosphonométhyl-butanedioïque, l'acide amino-éthane diphosphonique, l'acide diméthylamino-méthyl diphosphonique, l'acide éthylène diamine tétra méthylène phosphonique et l'acide propane-3-amino-1-hydroxy-1,1 diphosphonique, de préférence l'acide 1-hydroxyéthylidène-1,1 diphosphonique.

6. Procédé selon l'une quelconque des revendications précédentes, dans lequel le perrhénate radioactif contient soit du rhénium 186, soit du rhénium 188.

7. Procédé selon l'une quelconque des revendications précédentes, **caractérisé en ce que** la réaction est effectuée à un pH compris entre 2 et 2,5.

8. Procédé selon l'une quelconque des revendications précédentes, **caractérisé en ce que** la réaction est effectuée par chauffage desdits composants de la réaction à une température d'approximativement 120 °C.

9. Procédé de préparation d'une composition radiopharmaceutique stérile comprenant une solution d'un complexe de rhénium radioactif, **caractérisé en ce que** ladite solution de complexe de rhénium radioactif est préparée selon l'une quelconque des revendications précédentes et **en ce qu'**ensuite le pH du produit obtenu est ajusté à 5-6 par adjonction d'une solution tampon appropriée sous atmosphère inerte, tout en ajoutant si cela est souhaité un liquide de dilution pharmaceutiquement acceptable, après quoi la composition radiopharmaceutique finale est stérilisée par passage en autoclave à approximativement 120 °C et est par la suite stockée pendant une période d'au moins 3 heures avant administration.

10. Procédé de préparation d'une composition radiopharmaceutique stérile comprenant une solution d'un complexe de rhénium radioactif, **caractérisé en ce que** ladite solution de complexe de rhénium radioactif est préparée selon l'une quelconque des revendications 1 à 7 et **en ce que** le produit obtenu est passé en autoclave à approximativement 120 °C, après quoi le pH du produit stérile est ajusté à 4-9, de préférence à 5-8, par adjonction d'une solution tampon appropriée sous atmosphère inerte, tout en ajoutant si cela est souhaité un liquide de dilution pharmaceutiquement acceptable.

11. Procédé de préparation d'une composition radiopharmaceutique stérile comprenant une solution d'un complexe de rhénium radioactif, **caractérisé en ce que** ladite solution de complexe de rhénium radioactif est préparée selon la revendication 8 et **en ce qu'**ensuite le pH du produit obtenu est ajusté à 4-9, de préférence à 5-8, par adjonction d'une solution tampon appropriée sous atmosphère inerte, tout en ajoutant si cela est souhaité un liquide de dilution pharmaceutiquement acceptable.

12. Composition radiopharmaceutique stérile pour application thérapeutique, comprenant le produit préparé conformément au procédé selon l'une quelconque des revendications 9 à 11.

13. Utilisation d'une composition selon la revendication 12 pour la fabrication d'un médicament permettant de combattre ou de maîtriser des tumeurs ou permettant d'atténuer la douleur provoquée par certaines tumeurs métastatiques.

14. Trousse de préparation d'une composition radiopharmaceutique stérile pour application thérapeutique, comprenant (i) une solution d'un complexe de rhénium radioactif stérile obtenue par exécution du procédé selon l'une quelconque des revendications 1 à 7, puis par le passage en autoclave du produit ainsi obtenu à approximativement 120 °C, ou par exécution du procédé selon la revendication 8, et (ii) une solution tampon appropriée pour ajuster le pH de la solution définie en (i) à 4-9, de préférence à 5-8, à laquelle solution, si cela est souhaité, peut être ajouté un liquide de dilution pharmaceutiquement acceptable.
